(19) Europäisches Patentamt
European Patent Office
Office européen des brevets

(11) **EP 4 252 813 A1**

(12) **EUROPEAN PATENT APPLICATION**

(43) Date of publication:
**04.10.2023 Bulletin 2023/40**

(21) Application number: **22169788.1**

(22) Date of filing: **25.04.2022**

(51) International Patent Classification (IPC):
**A61M 16/10** (2006.01)   **A61M 16/12** (2006.01)
**A61M 16/06** (2006.01)   **A61M 16/00** (2006.01)
**A61M 16/20** (2006.01)

(52) Cooperative Patent Classification (CPC):
**A61M 16/026; A61M 16/0069; A61M 16/0677;**
**A61M 16/1005; A61M 16/125; A61M 16/204;**
A61M 16/0066; A61M 2016/0027; A61M 2016/0039;
A61M 2016/1025; A61M 2202/0208;
A61M 2205/3317; A61M 2205/3334;
A61M 2205/3344; A61M 2205/50;   (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **30.03.2022   US 202263325189 P**

(71) Applicant: **Koninklijke Philips N.V.**
**5656 AG Eindhoven (NL)**

(72) Inventors:
• **BRANS, Harold Johannes Antonius**
**Eindhoven (NL)**
• **BOU JAWDE, Samer**
**Eindhoven (NL)**
• **DE GRAAF, Pascal**
**Eindhoven (NL)**

(74) Representative: **Philips Intellectual Property &**
**Standards**
**High Tech Campus 52**
**5656 AG Eindhoven (NL)**

(54) **HIGH FLOW NASAL THERAPY SYSTEM AND METHOD**

(57)   A therapy system has a nasal cannula patient interface configured to deliver gas to a nasal cavity of a patient and a delivery system for delivering oxygen-enriched breathing gas, comprising air and enrichment oxygen, to the patient interface. The delivery system comprises a blower. An electrical power consumption of the blower is monitored and the timing of inhalation and exhalation is derived from the monitored electrical power. The delivery system is then controlled in dependence on the determined timing of inhalation and exhalation.

FIG. 1

60

Power
Monitoring

68

Level of Increase/
Decrease

62

Increase/
Decrease

70

Patient effort

64

IE detection

66

RR

72

HFNT settings
(flow and FiO2)

FIG. 4

(52) Cooperative Patent Classification (CPC): (Cont.)
A61M 2230/205

**Description**

FIELD OF THE INVENTION

This invention relates to High Flow Nasal Therapy (HFNT).

BACKGROUND OF THE INVENTION

HFNT provides high flows (compared to conventional flows) to patients through a nasal cannula. The basic idea is an extension to traditional oxygen support. Oxygen therapy typically provides only oxygen at low rates that usually range from 1 to 15 L/min. The oxygen is delivered via a so-called patient interface, which may comprise a nasal cannula, a face mask, a venturi mask, or a reservoir bag mask.

HFNT instead has two high pressure sources, for an air flow and for an oxygen flow. In combination, these high flows can reach a total of 60 L/min and more.

HFNT therapy is delivered through a device that has two main control functions, comprising the total rate of gas flow and the percentage of oxygen being delivered. The oxygen support is provided because HFNT was initially introduced as an elevated therapy for adults already on oxygen therapy (at normal flow rates) and for patients who are most likely hypoxemic or suffer from hypoxemic respiratory failure.

The basic device includes an air/oxygen blender and a heated humidifier to make sure the air is delivered at the appropriate temperature and humidity to the patient, mainly for comfort. With the blender, the fraction of inspired oxygen (FiO2) is for example controllable to range from 21% to 100%.

The HFNT device is reported as offering several potential benefits, including:

maintenance of a constant FiO2;
generation of a positive end-expiratory pressure (PEEP) and increased end-expiratory lung volume;
reduction of the anatomical dead space;
improvement of mucociliary clearance; and
reduction in the breathing frequency and work of breathing

Additionally, only requiring a nasal cannula instead of a mask means that HFNT provides much more comfort to the patients and allows them to perform more naturally their daily activities (e.g. eating, drinking, etc.).

Unfortunately, the primary use of HFNT is limited to stable hypoxemic patients, which restricts it to a limited patient population and also mainly restricts it to in-hospital usage. HFNT does not provide control over the pressure to allow for therapies that benefit other patients, e.g. hypercapnic patients.

The invention is based on the recognition that the potential of HFNT therapy is not utilized, such as the ability to provide dead space washout and CO2 clearance and a reduction in work of breathing.

One way to extend the range of therapy options is to detect the breathing cycle of the patient, and hence detect the inhalation and exhalation (IE) state. Currently, detection of the IE state of a patient is done by measuring the pressure as close as possible to the patient interface. By monitoring the pressure, an estimation can be derived for the IE state. By breathing, the patient influences the peak flow of the device which causes pressure differences. Monitoring these differences is not straightforward, and the quality of the obtained data suffers due to a low signal to noise ratio and due to air leakages.

The IE state derived from pressure measurements is also not truly a real-time measurement. Instead, the IE state at time t is a prediction obtained from data at time t-x. A real time measurement would be more effective, as it would better reflect the current respiratory state of the patient.

With accurate knowledge of the IE state of the patient, parameter settings can be tailored more accurately during therapy, to meet the needs of the patient at that moment.

US 2019/217030 discloses a high flow therapy system in which a breathing cycle is determined so that a flow can be delivered with a phase matching that of the patient's breathing cycle. A blower motor speed is one parameter which may be used to detect the breathing cycle.

SUMMARY OF THE INVENTION

The invention is defined by the claims.

According to a first aspect of the invention, there is provided a therapy system, comprising:

a nasal cannula patient interface configured to deliver gas to a nasal cavity of a patient;
a delivery system for delivering oxygen-enriched breathing gas, comprising air and enrichment oxygen, to the patient interface and comprising a blower;
a power monitoring system for monitoring an electrical power consumption of the blower; and
a controller which is adapted to:

determine the timing of inhalation and exhalation from the monitored electrical power; and
control the delivery system in dependence on the determined timing of inhalation and exhalation.

The invention is based on the recognition that the power of the blower can be used to detect the transitions between inhalation and exhalation. The blower power is an indirect parameter which is related to the inhalation and exhalation state of the patient. The power may be monitored by sensing current (e.g. for a constant voltage system) or by sensing voltage (for a constant current system) or by sensing current and voltage. The controller can monitor the blower power for different combinations of accessories (such as different tubing configurations, nasal cannula, gas supply system). The therapy settings can then be adapted based on the timing of the breathing cycle.

An advantage of monitoring blower power is that the power will vary depending on load even for a constant rotational speed. Thus, the blower power depends on the load and thus dynamic follows breathing. The blower rotational speed is for example in some HFNT system fixed, so that the rpm cannot be used to monitor IE, whereas the power consumption is not controlled. Thus, when there is a higher load such as during exhalation, even if there is a fixed blower rpm, the power to achieve that fixed rpm will be higher than at a lower load condition such as during inhalation. Thus monitoring power is provides a representative measure for all types of HFNT control.

The blower power signal may be used in combination with a pressure sensing signal to make more accurate estimates of inhalation/exhalation ("IE"). For example, a reduced pressure with reduced blower power signifies start of inhalation.

The system may further comprise a pressure sensor arrangement to enable measurement or estimation of a pressure at the nasal cavity. This then enables controlled pressure support to be provided.

The controller is for example adapted to control the delivery system to deliver a target total flow and a target oxygen percentage, and to regulate the pressure during each breath.

The HFNT therapy system (i.e. with oxygen delivery and air delivery to a nasal cannula) is thereby enhanced to provide control of the pressure support provided across the breathing cycle, and further taking account of the timing of inhalation and exhalation.

The pressure control is achieved by continuously sensing a pressure value (or sensing other parameters from which pressure can be derived), for example, at the nostrils, within the cannula, inside the mouth, or at the device just before the cannula. The delivery system is then controlled to obtain a set target pressure for example by altering the air flow (i.e. the gas delivery flow rate).

The control of the pressure support may be varied across the breathing cycle i.e. inhalation and exhalation. This enables the system to provide better therapy not only for hypoxemic patients but also to provide the benefits of comfort and simplicity of HFNT to other patient populations such as COPD and OSA patients, both of which have much higher prevalence and dominance in the population (in comparison to stable hypoxemic patients).

By providing this additional control over the parameters of HFNT, the therapy may be extended to provide a reliable treatment both at home and in hospital. For example, if the amount of pressure support (e.g. PEEP) is controlled then the device can be utilized at home for COPD or OSA patients instead of CPAP. This will provide the pressure support of a CPAP system, but with the advantage of the comfort of the HFNT approach. More importantly, as COPD is a major respiratory disease and patients are often treated through ventilators at home, this will greatly extend the patient population that can use HFNT.

The controller may be adapted to regulate the pressure between first and second pressure levels during each breath, to provide bi-level positive airway pressure control. The first pressure level is an IPAP pressure and the second pressure level is an EPAP pressure. Thus, BiPAP pressure control is enabled.

The pressure sensor arrangement for example comprises one or more of:

a pressure sensor at the nasal cavity;
a flow sensor for measuring flow to the nasal cavity; and
a pressure sensor for measuring a pressure at the delivery system.

Thus, various measures of pressure and flow may be used to measure, or enable estimation of, the nasal cannula pressure. The sensed parameters may be combined with data relating to the flow resistance of the various components of the system.

The controller may be adapted to end the delivery of enrichment oxygen by the delivery system at a time which is at or before the end of an inhalation phase.

Delivering oxygen during exhalation does not contribute to gas exchange, so oxygen can instead be conserved.

Delivering oxygen near the end of inhalation may also not be as useful as at the start of inhalation.

The controller may be adapted to increase the flow of air by the delivery system when the delivery of oxygen is ended. Thus, the overall flow is maintained, and hence the pressure control remains effective.

The controller may be further adapted to determine the respiratory rate from the timing of inhalation and exhalation. This provides a measure which may be used to monitor a patient condition over time.

The controller is for example adapted, in response to a determined raised respiratory rate, to adjust the delivery system to promote a reduction in respiratory rate. This then enhances relaxed breathing of the patient. The controller may also provide an indication of disease progression based on tracking of the respiratory rate. Some conditions result in progressive respiratory rate changes so they may be used as indication of disease severity.

The controller may be adapted to determine a patient breathing effort over time from an amplitude of the blower electrical power consumption over time. This patient effort can be compared with an expected breathing effort profile to detect differences, or changes in breathing effort over time may be tracked for disease progression monitoring.

The controller may also be adapted to determine if the patient has an open or closed mouth. This can again be used to control the therapy settings to provide the best results. This mouth open or closed condition is for example used to control PEEP of the patient during exhalation.

The invention also provides a computer implemented method of controlling a therapy system comprising a nasal cannula patient interface configured to deliver gas to a nasal cavity of a patient, a delivery system for delivering oxygen-enriched breathing gas, comprising air and enrichment oxygen, to the patient interface, the delivery system comprising a blower, wherein the method comprises:

> for monitoring an electrical power consumption of the blower;
> determining the timing of inhalation and exhalation from the monitored electrical power; and
> controlling the delivery system in dependence on the determined timing of inhalation and exhalation.

The method may comprise measuring or estimating a pressure at the nasal cavity, and controlling the delivery system to deliver a target total flow and a target oxygen percentage, and to regulate the pressure during each breath. The delivery of enrichment oxygen by the delivery system may be ended at a time which is at or before the end of an inhalation phase.

The method may also comprise determining the respiratory rate from the timing of inhalation and exhalation, and:

> in response to a determined raised respiratory rate, adjusting the delivery system to promote a reduction in respiratory rate; and/or
> providing an indication of disease progression based on tracking of the respiratory rate.

The invention also provides computer program comprising computer program code which is adapted, when said program is run on a computer, to implement the methods defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Figure 1 shows a therapy system for high flow nasal therapy;
> Figure 2 shows plots of the blower power, the airway flow and patient effort;
> Figure 3 shows a plot of blower power and the patient effort signal;
> Figure 4 shows how the power monitoring is used;
> Figure 5 shows the operating parameters of the system of Figure 1;
> Figure 6 shows pressure waveforms for the operation of the system of Figure 1;
> Figure 7 shows how the system of Figure 1 may be used in a simplified mode of operation to provide only pressure support; and
> Figure 8 shows the corresponding waveforms for the mode of operation of Figure 6.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a therapy system having a nasal cannula patient interface configured to deliver gas to a nasal cavity of a patient and a delivery system for delivering oxygen-enriched breathing gas, comprising air and enrichment oxygen, to the patient interface. The delivery system comprises a blower. An electrical power consumption of the blower is monitored and the timing of inhalation and exhalation is derived from the monitored electrical power. The delivery system is then controlled in dependence on the determined timing of inhalation and exhalation.

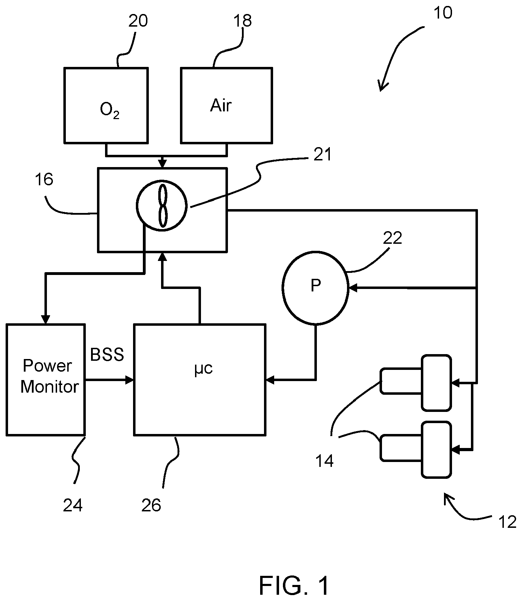

Figure 1 shows a therapy system 10 for high flow nasal therapy in schematic form.

The therapy system 10 comprises a nasal cannula patient interface 12 configured to deliver gas to a nasal cavity of a patient. The patient interface comprises a pair of nose prongs 14.

A delivery system 16 is provided for delivering oxygen-enriched breathing gas, comprising ambient air 18 and enrichment oxygen 20, to the patient interface 12. The delivery system for example comprises a flow pump (i.e. a blower 21) and a valve arrangement to control the coupling of ambient air and/or enrichment oxygen to the patient interface. It thus functions as an air/oxygen blender. The delivery system may also provide heating and humidification and/or dehumidification, not shown.

A pressure sensor arrangement 22 is provided to enable measurement or estimation of a pressure at the nasal cavity. It may comprise a pressure sensor, but other parameters such as flow rate may be monitored from which a pressure estimation may be derived.

A power monitor 24 is used to monitor the electrical power consumption of the blower 21 of the delivery system,. It thereby provides a breath sensor signal (BSS) which enables the timing of the different phases (inhalation, exhalation) of the breathing cycle to be determined.

A controller 26 controls the delivery system. In particular, it controls the delivery system to control pressure levels and/or enrichment oxygen levels at variable levels over time within the timing of each individual breathing cycle. In one option, a target total flow and a target oxygen percentage are provided (i.e. typical HFNT parameters) but additionally the delivery pressure is regulated so that single pressure CPAP control is enabled. Alternatively, the pressure may be regulated between first and second pressure levels during each breath, to provide bi-level positive airway pressure control. In another option, the controller is adapted to end the delivery of enrichment oxygen by the delivery system at a time which is at or before the end of an inhalation phase. The flow of air by the delivery system is for example increased when the delivery of oxygen is ended to maintain a desired flow rate. This reduces the amount of oxygen needed for HFNT therapy. The two approaches may be combined as part of a single therapy.

In addition to the new therapy approaches outlined above, the system may also be controllable to deliver existing conventional therapies, such as a first additional mode of operation with delivery of a target total flow and a target oxygen percentage without pressure regulation (i.e. conventional HFNT) and a second additional mode of operation in which the delivery system is controlled to deliver a target pressure without oxygen enrichment (i.e. conventional CPAP or bi-level PAP therapy). HFNT is conventionally always used with oxygen.

Currently, HFNT only controls the total delivered flow and the fraction of inspired oxygen (FiO2). HFNT can however be used to provide pressure support and it can be controlled within the time duration of each breath by having detection of the inhalation and exhalation timing, for example to enable bi-level pressure control. The pressure value which results from the control of flow depends on several factors but mainly the status of the mouth (open versus closed) and the total flow rate. A closed mouth and higher flow rates provide higher pressure levels.

The power monitor 24 is used to derive the timing of the breathing cycle by detecting changes which arise between inhalation and exhalation.

In particular, the patient breathing during HFNT is driven by their own respiratory effort, as the device delivers high flow through the nasal cannula. During inhalation, the blower of the delivery system is supported by the patient flow as the patient attempts to breath in. During exhalation, the opposite occurs. The blower is resisted by the patient flow as the patient attempts to exhale. This will change the rotary speed of the blower. Electronics in the blower module will correct for this and change the power until the revolution speed is at the correct level. Thus, there is a decrease in blower power during patient inhalation and an increase during exhalation. Therefore, by tracking the power consumed by the blower across time, the patient's respiratory effort can be indirectly tracked.

This power variation may be more evident than the change in blower speed, as a result of the regulation process explained above.

The power variation and the monitored pressure may be processed together to provide a more robust determination of the IE state of the patient.

As explained above, while motor speed can be a controlled parameter to obtain a required flow rate, the blower power is not, and this makes it particularly suitable for detecting the IE state. The motor power also provides a rapid detection of the load on the blower.

The inhalation/exhalation (IE) state can then be determined by looking at the pattern of the blower power variation. The transition from the exhalation phase to the inhalation phase is visible by a drop in power consumption. This is independent with respect to both the flow level of the HFNT device and the breathing effort of the patient. In other words, by observing the change at any given flow and patient effort, the increase and decrease in power consumption detects IE transition.

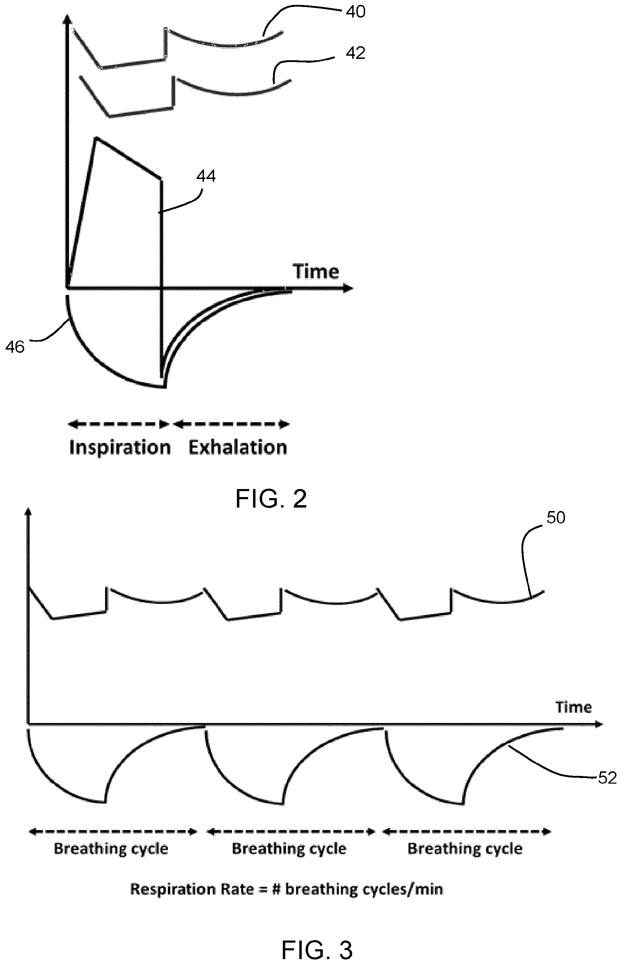

Figure 2 shows plots of the blower power (plot 40 is for a first flow rate, and plot 42 is for a lower, second flow rate) as well as the airway flow (plot 44) and patient effort (plot 46).

As the patient effort pulls in flow from the surroundings, the blower power required to deliver a constant flow decreases, allowing the detection of the inspiratory phase. The blower power is dependent on the HFNT flow setting. However, in all cases, and regardless of the variation in patient effort, a drop in power consumption signifies a transition from the exhalation phase to the inhalation phase.

By knowing the transition from the exhalation phase to the inhalation phase, the repeating cycle (respiratory rate, RR) is also known. When the blower power variation is measured in time, the RR can statistically be monitored to detect shortness of breath (dyspnea).

Figure 3 shows a plot of blower power consumption (plot 50), and the patient effort signal (plot 52). It shows that the respiration rate is clearly visible in the blower power trace. The blower power level also depends on the depth of breathing.

When for example the RR is too high, a possible adjustment to the therapy is to encourage the patient's breathing to return gradually to the target (or baseline) RR. The blower power cycle time will gradually be increased to fit the desired RR.

As explained above, with conventional HFNT, there are two main control parameters; air flow and FiO2. Currently, both need to be changed manually (by a Respiratory Therapist). There is no response by the HFNT device to an increasing RR. Theoretically, with an increased RR, the therapist could manually adjust both flow rate and oxygen flow. For example, they could increase the air flow and increase as well the oxygen percent delivered. This should reduce the RR to a normal baseline.

The therapy system of the invention may have a mode by which this control adaptation is performed automatically. For example, the therapist may set a baseline RR and the device will adjust to achieve that once the measured RR exceeds the baseline RR for a period time T (a number of hours) and a percentage X (e.g. 10%)

Accompanying an increase in blower cycle time,. other changes in the device settings such as the flow and FiO2 may also be adapted (e.g. increased) to further support the patient and reduce patient RR. Thus, this will also help in optimizing the therapy. Additionally, by tracking RR, the progression of the disease over time could be captured or an unexpected exacerbation of a COPD patient could be predicted, and intervention could be taken in advance.

The amplitude of the blower power consumption curve reveals information about the level of effort the patient applies during breathing as mentioned above. The lower the amplitude, the lower the effort. On the other hand, the shape of the curve shows if the breathing effort is balanced and fits the normal pattern. Similar to the therapy adjustment for RR, a "correct" blower power curve can be enforced to the HFNT device.

Similarly, by detecting the effort change over time, disease progression could be captured and/or HFNT settings could be adjusted.

A further last derivative from the blower power consumption is the possibility to detect if the respiratory patient has opened or closed his mouth during HFNT therapy. During exhalation with a closed mouth, the blower power will be higher than with an open mouth to achieve constant flow.

In order to control PEEP of the patient during exhalation there is a need to know if the mouth is closed or not. It is not possible to deliver any meaningful pressure when the mouth is open, so it has to be corrected another way. The device can for example alert the user of the amount of times the situation occurred after the user wakes up. The user could then opt to use a mouthpiece with a limited orifice to reduce leakage.

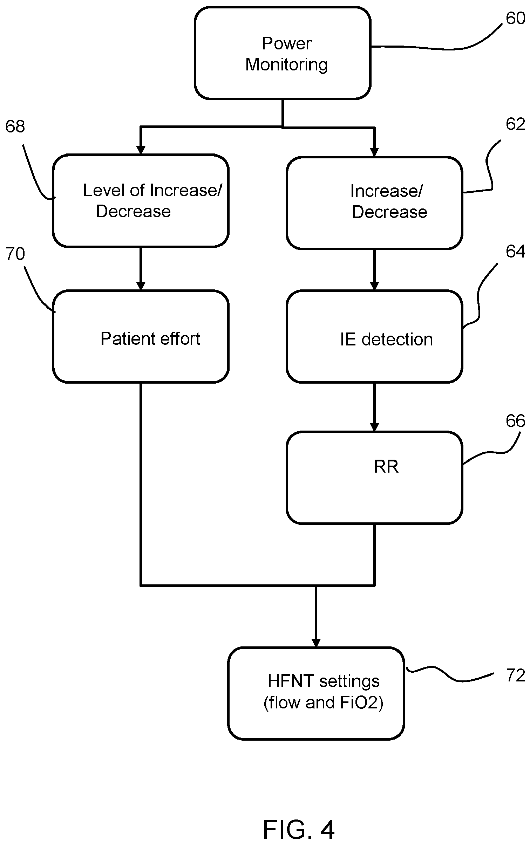

Figure 4 shows how the power monitoring is used. The power monitoring takes place in step 60.

In step 62, increases and decreases in power consumption are identified, and these enable detection of the transitions between inhalation and exhalation in step 64. The respiratory rate can then be detected in step 66, as well as trends in RR.

In step 68, the amplitude of the power consumption is monitored, and the amplitudes of changes power consumption so that a patient effort can be derived in step 70.

The information from both branches are used in step 72 to adapt the HFNT settings, such as the flow and FiO2.

Other sources of information may supplement the blower power measurement, for the detection of IE state. Current and/or voltage signals may be monitored as well as the blower revolution speed.

Monitoring multiple components (or elements) can also act as a quality control to ensure that the components function within their specification limits. For example, if one component shows a drift in output (compared to the other components), a specific alarm could be triggered with follow up actions (e.g., calibration, maintenance).

Some of the ways the IE timing information may be used will now be explained.

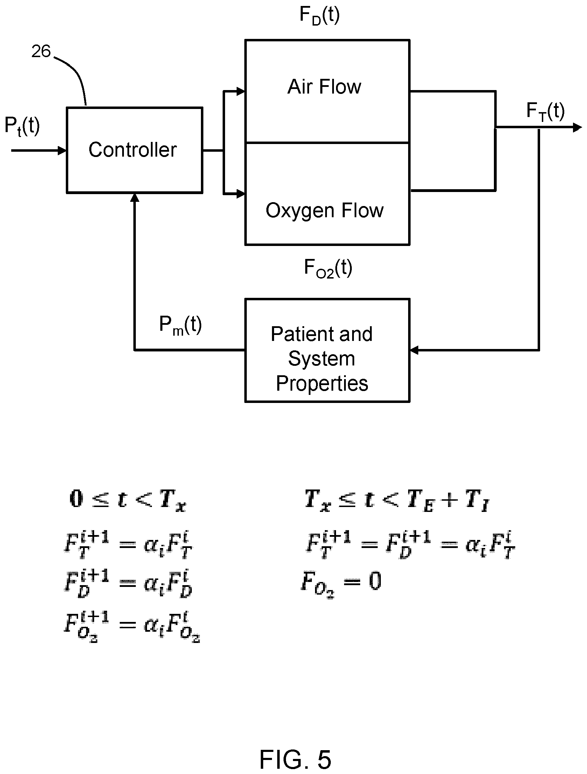

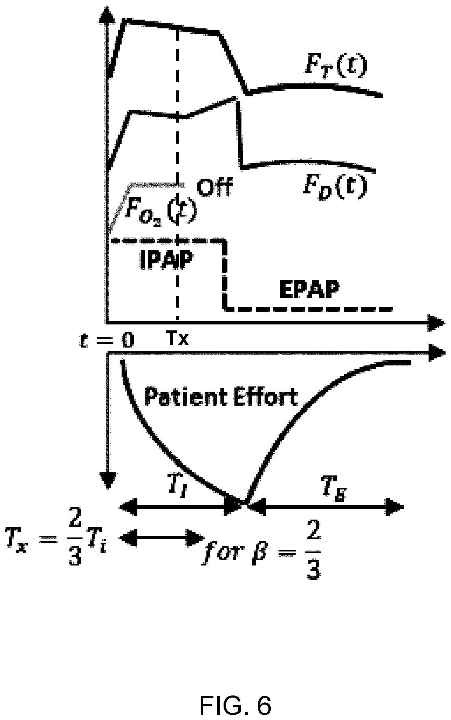

Figure 5 shows the operating parameters of the system of Figure 1 and Figure 6 shows corresponding pressure waveforms.

In a conventional HFNT system, the controlled variables are the total flow (i.e. the combined air and enrichment oxygen flow to the patient) and the FiO2. The dependent variables are the air flow (i.e. the air flow to the patient interface without the additional oxygen flow) and the oxygen flow. Note that the air flow could be sourced from compressed air (common in a hospital setting) or simply from the room with the aid of a blower (common in home settings). Regardless of the source, the same concepts apply. The oxygen flow comes from a compressed source in both cases.

In a first use of the IE timing information, the controlled variables are target pressures, for example a CPAP pressure, or an inspiratory PAP and an expiratory PAP to provide bi-level pressure control, the FiO2 and an upper flow limit. The dependent variables are the total flow, the air flow, the oxygen flow, total flow (oxygen + air flow) and the measured pressure value or bi-level pressure values.

Figure 5 shows the air flow as $F_D(t)$ and the oxygen flow as $F_{O2}(t)$ which combine to give the total flow $F_T(t)$.

A bi-level pressure control system will be described. The controller 26 receives a target pressure Pt(t) over time. A feedback system determines a measured pressure $P_m(t)$ which is used by the controller to set the air flow and oxygen flow.

By using the control approach shown in Figure 5, the HFNT system can behave additionally as a BiPAP device. A user may set two target pressures, the IPAP and the EPAP. For safety, the user can also set a maximum flow limit that the device should not exceed. Alternatively, this limit can be a built-in value over which the user has no control but is determined by the manufacturer.

The controller receives the measured pressure $P_m(t)$ and adjusts both the air flow and oxygen flow to achieve the target pressure and FiO2. The measured pressure will depend on the system properties (e.g. interface, cannula resistance) as well as the patient characteristics (e.g. respiratory resistance, breathing cycle, mouth open, mouth closed etc.).

The pressure could be a measured pressure value at the level of the nasal prongs or an estimated pressure at the nasal prongs through modelling the system behavior (e.g. estimating pressure at the nasal prongs by having the device pressure, flow, and the system properties related to the resistance of the cannula).

Figure 5 includes equations showing that the flow levels $F_T$, $F_D$ and $F_{O2}$ are controlled in an iterative way, wherein each is multiplied by a factor $\alpha_i$ at each iteration i.

The equations also show an further option, wherein after time $T_x$ (which is a fraction of the way through the inhalation phase of each breath cycle), the enrichment oxygen flow $F_{O2}$ is reduced to zero but the total flow is kept the same and hence fully provided by the air flow.

The parameter $\alpha$ may be set by the manufacturer or by the user through an advanced user setting. It captures the change (increase or decrease) between the current flow delivered by the device and the new flow that will help achieve the target pressure. There could be several methods to set $\alpha$, at each iteration step (*i*).

One possible simple equation is given by:

$$\alpha(i) = \alpha_i = 1 - \frac{P_m - P_t}{P_t} \qquad \alpha_{min} \leq \alpha_i \leq \alpha_{max}$$

Where $P_m$, $P_t$, $\alpha_{min}$, and $\alpha_{max}$ represent the measured pressure, target pressure, minimum $\alpha$ and maximum $\alpha$ values.

For illustration, it is assumed that the flow is 30L/min, the target pressure is 10 cmH2O (10 cmH2O =9.8 Pa), $\alpha_{max}$ is 1.2 and $\alpha_{min}$ is 0.8.

The measured pressure may be assumed to be 8 cmH2O (7.8 Pa) and thus less than the target pressure. In this case, $\alpha$ is equal to exactly $\alpha_{max}$ [1-(8-10)/10]. The device will increase the total flow by 20% or by 1.2 to 36 L/min (30*1.2).

The new measured pressure increases to 11 cmH2O (10.8Pa) and now is larger than the target pressure. The new value of $\alpha$ is now 0.9 [1-(11-10)/10] which falls within the range. The new flow will become 32.4 L/min (36*0.9). Now the measured pressure is 10.5 cmH20 (10.3 Pa) and is getting closer to target pressure as the process continues. Furthermore, as explained above, it is possible to limit the delivery of oxygen to conserve it as well as efficiently using it. Delivering oxygen during patient exhalation does not contribute to gas exchange. Also, delivering oxygen towards the end of inhalation might not be as useful as oxygen delivered at the beginning of inhalation. Thus, it is possible to set a time $T_x$ (measured from the onset of inhalation, patient effort, or *t* = 0) that could be equal to or less than the inhalation time $T_i$ ($T_x = \beta T_i$ *where* $0 < \beta < 1$) to achieve that objective. The value of $\beta$ could be set by the manufacturer, or selected by the therapist based on need or observation such as for example based on monitoring SpO2 values.

After the time $T_x$ has passed, the oxygen delivered flow is turned off ($F_{O_2}$ = 0) and the loss in the total flow ($F_T$) is compensated with an increase by the air flow ($F_D$). Figure 6 shows an illustration how the various flows (oxygen, device, and total) will vary across a patient effort curve for and BiPAP pressure control.

The oxygen flow stops at $T_x$ and the air flow increases to compensate, maintaining a stable total flow. Additionally, during inhalation, the total flow required to maintain the required pressure level of the bi-level control is larger than during exhalation due to the patient effort.

The control approach of Figures 5 and 6 makes use of a time value Tx which is a fraction of the inhalation time duration Ti. Thus, the inhalation time duration needs to be known at the beginning of the inhalation period, or the patient effort timing needs to be known. This is the purpose of the power monitor 24.

The inhalation time duration Ti could be continuously estimated from several previous cycles (e.g. from the previous 50 breaths). The inhalation time duration can be obtained by detecting the transition from inhalation to exhalation.

While delivering oxygen flow has been an essential element in HFNT, the ability to provide pressure support by itself is useful enough to be used in patients who are not hypoxemic but in need of pressure support (i.e. CPAP or BiPAP). Thus, this decouples the current classical notion regarding the coupling between oxygen support and HFNT allowing the latter to have wider applications.

Increasing the pressure will further expand and recruit the lung units allowing more gas exchange to occur. This will then increase the amount of oxygen in the blood and thus the SpO2 level. Thus since the level of SpO2 depends on the pressure which itself depends on the flow by varying the flow, the system can also be used to control the SpO2 of patients.

The incremental change in pressure explained above (using value $\alpha$) applies to a baseline $\alpha$ value that can achieve the designated pressure(s). In other words, rather than abruptly changing flow to reach the desired target, this mechanism comfortably, safely, and gradually changes the flow rates to reach the desired overall pressure level and flow settings. The adaptation using the value $\alpha$ could occur during the initial setup across a breath or across a couple of breath in order to reach an estimated baseline. However, once set-up, and during the breath cycle itself, the switching between flow levels to achieve and maintain the IPAP and EPAP pressures is as near to instantaneous as possible (as can be seen in the traces for $F_D(t)$ and $F_T(t)$ in Figure 6).

Thus, once the system has reached a required overall operating point with an estimated baseline $\alpha$, the pressure levels basically toggle between the same two values (one for inhalation and one for exhalation) at each successive breath by instantaneous flow variation.

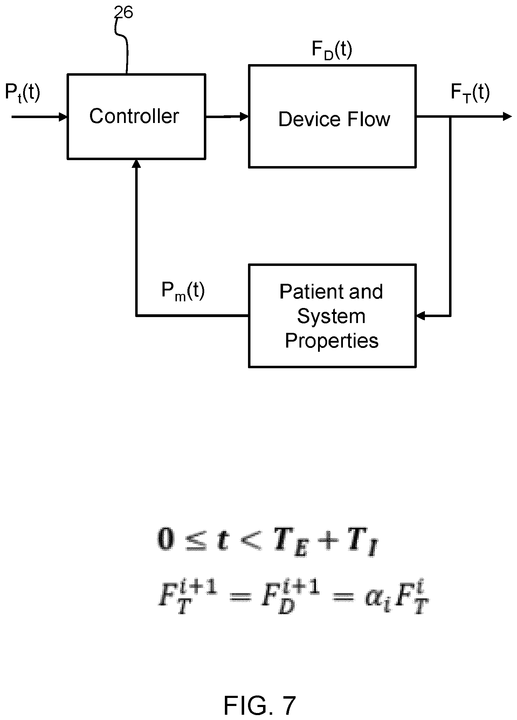

Figure 7 shows how the system may be used to provide only pressure support. In this case, the controller 26 only controls the air flow $F_D(t)$ which is thus equal to the total flow $F_T(t)$. This simplifies the process of the controller since it now only has one source of flow.

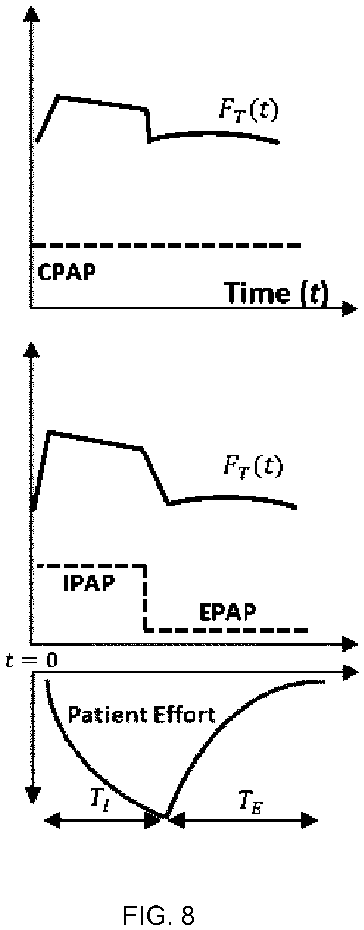

Figure 8 shows the corresponding waveforms.

The system could also be used to detect when the nasal cannula is not properly detected or totally disconnected. In case the total flow delivered is constantly increasing (e.g. this can be detected if $\alpha$ is not changing and being set to the maximum value at every iteration for a considerable time (e.g. 5-10 minutes while the pressure measured is minimally changing), then a warning could be provided to the user to properly set the nasal cannula or put it on. In the case of no response or change in few minutes, then most probably the patient is not using the device and it could be turned off automatically.

To avoid any pressures beyond physiologically accepted levels, or pressures that could be high for specific patients (as determined by a patient's clinician for example), the device could have an additional pressure safety to avoid reaching this pressure. This could be done by reducing the flow or limiting the flow to the values that do not lead to the pressure being exceeded.

The application has wide use both at home and in hospitals/clinics. It is of particular interest for COPD and OSA patients and particularly mild to moderate cases in need of pressure support in addition to stable hypoxemic patients.

The description above refers to High Flow Nasal Therapy, HFNT. It is however noted other terms are also used to describe the same therapy or device, such as high flow nasal cannula (HFNC), nasal high flow (NHF) or high flow nasal oxygen (HFNO). The term HFNT is intended to cover all of these different therapies and devices.

The concept explained above can be extended to other ventilation modes in which the patient is not fully sedated and actively breathing (e.g. pressure support ventilation mode). In those cases, the method can help detect IE and support the ventilator in triggering a breath. The accuracy of the IE detection as explained above avoid s patient-ventilator asynchrony.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1.  A therapy system, comprising:

    a nasal cannula patient interface (12) configured to deliver gas to a nasal cavity of a patient;
    a delivery system (16) for delivering oxygen-enriched breathing gas, comprising air and enrichment oxygen, to the patient interface and comprising a blower;
    a power monitoring system (24) for monitoring an electrical power consumption of the blower; and
    a controller (26) which is adapted to:

       determine the timing of inhalation and exhalation from the monitored electrical power; and
       control the delivery system in dependence on the determined timing of inhalation and exhalation.

2.  The system of claim 1, further comprising a pressure sensor arrangement to enable measurement or estimation of a pressure at the nasal cavity.

3.  The system of claim 2, wherein the controller is adapted to control the delivery system to deliver a target total flow and a target oxygen percentage, and to regulate the pressure during each breath.

4.  The system of any one of claims 2 to 3, wherein the pressure sensor arrangement (22) comprises one or more of:

       a pressure sensor at the nasal cavity;
       a flow sensor for measuring flow to the nasal cavity;
       a pressure sensor for measuring a pressure at the delivery system.

5.  The system of any one of claims 1 to 4, wherein the controller is adapted to end the delivery of enrichment oxygen by the delivery system at a time which is at or before the end of an inhalation phase.

6.  The system of claim 5, wherein the controller is adapted to increase the flow of air by the delivery system when the delivery of oxygen is ended.

7.  The system of any one of claims 1 to 6, wherein the controller is further adapted to determine the respiratory rate from the timing of inhalation and exhalation.

8.  The system of claim 7, wherein the controller is adapted to:

       in response to a determined raised respiratory rate, adjust delivery system to promote a reduction in respiratory rate; and/or
       provide an indication of disease progression based on tracking of the respiratory rate.

9.  The system of any one of claims 1 to 8, wherein the controller is adapted to determine a patient breathing effort over time from an amplitude of the blower electrical power consumption over time.

10. The system of any one of claims 1 to 9, wherein the controller is adapted to determine if the patient has an open or closed mouth.

11. A computer implemented method of controlling a therapy system comprising a nasal cannula patient interface configured to deliver gas to a nasal cavity of a patient, a delivery system for delivering oxygen-enriched breathing gas, comprising air and enrichment oxygen, to the patient interface, the delivery system comprising a blower, wherein the method comprises:

for monitoring an electrical power consumption of the blower;
determining the timing of inhalation and exhalation from the monitored electrical power; and
controlling the delivery system in dependence on the determined timing of inhalation and exhalation.

12. The method of claim 11, comprising measuring or estimating a pressure at the nasal cavity, and controlling the delivery system to deliver a target total flow and a target oxygen percentage, and to regulate the pressure during each breath.

13. The method of claims 11 or 12, comprising ending the delivery of enrichment oxygen by the delivery system at a time which is at or before the end of an inhalation phase.

14. The method of any one of claims 11 to 13, comprising determining the respiratory rate from the timing of inhalation and exhalation, and:

in response to a determined raised respiratory rate, adjusting the delivery system to promote a reduction in respiratory rate; and/or
providing an indication of disease progression based on tracking of the respiratory rate.

15. A computer program comprising computer program code which is adapted, when said program is run on a computer, to implement the method of any one of claims 11 to 14.

FIG. 1

Inspiration   Exhalation

FIG. 2

Breathing cycle   Breathing cycle   Breathing cycle

Respiration Rate = # breathing cycles/min

FIG. 3

FIG. 4

$F_D(t)$

26

$P_t(t)$ → Controller

Air Flow

Oxygen Flow

$F_T(t)$

$F_{O2}(t)$

$P_m(t)$

Patient and System Properties

$$0 \leq t < T_x \qquad\qquad T_x \leq t < T_E + T_I$$

$$F_T^{i+1} = \alpha_i F_T^i \qquad\qquad F_T^{i+1} = F_D^{i+1} = \alpha_i F_T^i$$

$$F_D^{i+1} = \alpha_i F_D^i \qquad\qquad F_{O_2} = 0$$

$$F_{O_2}^{i+1} = \alpha_i F_{O_2}^i$$

FIG. 5

FIG. 6

26

$P_t(t)$ → | Controller | → | Device Flow | → $F_T(t)$

$F_D(t)$

$P_m(t)$ ← | Patient and System Properties |

$$0 \leq t < T_E + T_I$$

$$F_T^{i+1} = F_D^{i+1} = \alpha_i F_T^i$$

FIG. 7

FIG. 8

Europäisches
Patentamt

European
Patent Office

Office européen
des brevets

## EUROPEAN SEARCH REPORT

Application Number

EP 22 16 9788

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/173219 A1 (RESMED MOTOR TECHNOLOGIES INC) 21 November 2013 (2013-11-21) | 1-10,15 | INV. A61M16/10 A61M16/12 |
| A | * figures 1-8 * * paragraph [0027] * | 11-14 | A61M16/06 A61M16/00 A61M16/20 |
| X | US 2016/193438 A1 (WHITE CRAIG KARL [NZ] ET AL) 7 July 2016 (2016-07-07) | 1-10,15 | |
| A | * figures 1-38 * * paragraph [0558] - paragraph [0910] * | 11-14 | |
| X | US 5 740 795 A (BRYDON JOHN WILLIAM ERNEST [AU]) 21 April 1998 (1998-04-21) | 1-10,15 | |
| A | * figures 1-3 * * column 2, line 56 - column 3, line 17 * | 11-14 | |
| X | US 2006/196508 A1 (CHALVIGNAC PHILIPPE [FR]) 7 September 2006 (2006-09-07) | 1-10,15 | |
| A | * figures 1-9 * * paragraph [0157] - paragraph [0169] * | 11-14 | TECHNICAL FIELDS SEARCHED (IPC) |
| A | US 10 799 663 B1 (AIRES MEDICAL LLC [US]) 13 October 2020 (2020-10-13) * figures 1I-M * * paragraph [0051] - paragraph [0089] * | 1-15 | A61M |

The present search report has been drawn up for all claims

| Place of search | Date of completion of the search | Examiner |
|---|---|---|
| Munich | 14 October 2022 | Liess, Helmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 9788

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2022

| Patent document cited in search report | | Publication date | Patent family member(s) | | Publication date |
|---|---|---|---|---|---|
| WO 2013173219 | A1 | 21-11-2013 | AU | 2013263072 A1 | 06-11-2014 |
| | | | CN | 104486994 A | 01-04-2015 |
| | | | EP | 2849643 A1 | 25-03-2015 |
| | | | JP | 6396892 B2 | 26-09-2018 |
| | | | JP | 2015516266 A | 11-06-2015 |
| | | | JP | 2018083112 A | 31-05-2018 |
| | | | NZ | 630627 A | 29-07-2016 |
| | | | US | 2015136136 A1 | 21-05-2015 |
| | | | US | 2022062570 A1 | 03-03-2022 |
| | | | WO | 2013173219 A1 | 21-11-2013 |
| US 2016193438 | A1 | 07-07-2016 | AU | 2014316671 A1 | 24-03-2016 |
| | | | AU | 2019275640 A1 | 02-01-2020 |
| | | | AU | 2021203998 A1 | 08-07-2021 |
| | | | EP | 3030302 A1 | 15-06-2016 |
| | | | US | 2016193438 A1 | 07-07-2016 |
| | | | WO | 2015033288 A1 | 12-03-2015 |
| US 5740795 | A | 21-04-1998 | DE | 69425113 T2 | 16-11-2000 |
| | | | EP | 0656216 A2 | 07-06-1995 |
| | | | US | 5740795 A | 21-04-1998 |
| US 2006196508 | A1 | 07-09-2006 | AT | 554815 T | 15-05-2012 |
| | | | AU | 2004208575 A1 | 12-08-2004 |
| | | | CA | 2518308 A1 | 12-08-2004 |
| | | | CN | 1761498 A | 19-04-2006 |
| | | | EP | 1590028 A1 | 02-11-2005 |
| | | | FR | 2850284 A1 | 30-07-2004 |
| | | | TW | I332850 B | 11-11-2010 |
| | | | US | 2006196508 A1 | 07-09-2006 |
| | | | US | 2013019869 A1 | 24-01-2013 |
| | | | WO | 2004067070 A1 | 12-08-2004 |
| US 10799663 | B1 | 13-10-2020 | US | 10874817 B1 | 29-12-2020 |
| | | | US | 2020179638 A1 | 11-06-2020 |
| | | | US | 2020306486 A1 | 01-10-2020 |
| | | | US | 2021196918 A1 | 01-07-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 2019217030 A